# EUROPEAN PATENT SPECIFICATION

(11) **EP 0 284 227 B1**
(45) Date of publication and mention of the grant of the patent: **28.07.1993**
(21) Application number: 88301879.8
(22) Date of filing: 03.03.1988
(51) Int. Cl.: B01D 53/04, A61M 16/06

(54) **Apparatus and process for the recovery of halogenated hydrocarbons in a gas stream**
Vorrichtung und Verfahren für die Wiedergewinnung von halogenierten Kohlenwasserstoffen in einem Gasstrom
Installation et procédé pour la récupération d'hydrocarbures halogénés dans un courant gazeux

(30) Priority: 04.03.1987 CA 531133
(43) Date of publication of application: 28.09.1988
(73) Proprietor: PRAXAIR CANADA INC., Mississauga, Ontario, L5B 1M2 (CA)
(72) Inventor: Filipovic, Dusanka, Ontario (CA); Sweatman, Fraser, Toronto Ontario (CA)
(74) Representative: Lerwill, John

(56) References cited:
- DE-B- 2 214 153
- FR-E- 86 888
- US-A- 3 867 936
- US-A- 3 941 573
- US-A- 4 104 294
- Ullmanns Encyklopädie der technischen Chemie, 4th edition, volume 2, 1972, pages 610-619

## Description

### FIELD OF THE INVENTION

This invention relates to the recovery of halogenated hydrocarbons from a gas stream and recovery thereof for reuse.

### BACKGROUND OF THE INVENTION

Halogenated hydrocarbon compounds include the family of compounds of bromo-, fluoro- and/or chloroethers, fluorinated alkyl ethers, chlorofluorocarbons and chlorofluoro ethers and their derivatives. This family of compounds are typically used as solvents, refrigerants, anesthetic gases, aerosol propellants, blowing agents and the like. Many of these compounds are widely used and normally discharged into the atmosphere. However, if these compounds could be recovered and re-used there would be a considerable cost saving and reduction in environmental pollution. In view of the possible effects of released anesthetic gases, attempts have already been made to recover such gases.

An example of anesthetic gas removal, is with regard to patient exhalent to ensure that the environment in the operating theatre does not contain anesthetic gases which can have a long term effect on the professionals conducting the operation. Commonly, anesthetic gases are removed from patient exhalent by use of various types of disposable absorbers, such as that disclosed in US-A-3,867,936 and US-A-3,941,573. In the United States patent to Kelley, US-A-3,867,936, an absorber unit is in the shape of a hollow drum filled with activated carbon to absorb anesthetic gases exhaled by the patient. When the weight of the absorber unit increases to a predetermined value, the unit is replaced with a fresh one. In Chapel, US-A-3,941,573, a molecular sieve is used in combination with the activated carbon in a disposable cartridge. The cartridge is included in the patient anesthetic administration breathing system to absorb on both the activated carbon and the molecular sieve materials the exhaled anesthetic gases.

It is common to dispose of the absorber units used to absorb anesthetic gases. However, in view of the rising costs of the anesthetic gases, attempts are being made to recover them. For example, in US-A-3,592,191, a system is provided for recovering exhausted anesthetic gases from patient exhalent by removing water vapor from the collected gases by their condensation thereof or with a hygroscopic material. This treated gas then has the anesthetic agent extracted therefrom by a cryogenic process in which the vapors of the anesthetic gases are condensed to liquid phase, or by removal on an absorbent material which is processed later to remove the anesthetic agents. The collected anesthetic liquids are then reintroduced directly into the anesthetic system. Such approach has little if any facility to control bacterial contamination and recycle of harmful microorganisms to the patient.

Another approach in the recapture of anesthetic gases is disclosed in CS-B-185,876. An absorbent material is used to absorb halogenous inhalant anesthetics from the patient exhalent. When the adsorbent material is saturated, it is removed in an appropriate container and placed in a regeneration system. A purging gas, such as steam, is used to remove the anesthetic agents from the adsorbent material. The purged gas is then collected with water removed therefrom and the separated anesthetic agents are subjected to fractionation to separate out the individual anesthetic agents from the supply of anesthetic gases from various operating theatres.

The use of molecular sieves to adsorb gaseous components is exemplified in US-A-3,729,902. Carbon dioxide is adsorbed on a molecular sieve which is regenerated with heated steam to remove the carbon dioxide from the adsorbent material. Another example of the use of molecular sieves to adsorb organic materials is disclosed in CA-A-1,195,258. In this instance, a hydrophobic molecular sieve is used to adsorb organic species from a gas stream containing moisture. The hydrophobic molecular sieve selectively adsorbs the organic molecular species into the adsorbent material, while preventing the collection of water vapour from the gas stream on the adsorbing material. The temperature and pressure at which the system is operated is such to prevent capillary condensation of the water in the gas stream onto the adsorbing material. By removing the adsorbing material from the system, the adsorbing material is essentially free of water yet has absorbed thereon the desired organic molecular species. The organic molecular species are then recovered from the adsorbent material by purging.

Another molecular sieve material is known from US-A-4104294, which discloses crystalline silicates essentially free of aluminium oxides.

Particularly desirable types of anesthetic gases are commonly sold under the trade marks ETHRANE and FORANE as disclosed in US-A-3,469, 011; US-A-3,527,813; US-A-3,535,388; and US-A-3,535,425. These types of anesthetic gases are particularly expensive; hence an effective method of recovering them from patient exhalent for reuse would be economically advantageous.

### SUMMARY OF THE INVENTION

According to an aspect of the invention, there is provided a process for the recovery of at least one halogenated hydrocarbon from a gas stream which also includes water vapours, comprising the steps of passing the gas stream through a bed of an adsorbent material to adsorb the halogenated hydrocarbon into the bed with minimal adsorption of water vapour, and removing the bed of adsorbent material containing the adsorbed halogenated hydrocarbon from the gas stream;
characterised in that the adsorbent material consists essentially of a high silica zeolite material having a ratio of SiO₂/Al₂O₃ of at least in the range of 12 to 50, the bed having pore diameters large enough to permit molecules of the halogenated hydrocarbon to pass therethrough for selective adsorption in the larger internal cavities of the crystal framework; and that said bed of absorbent material is regenerated by exposing it to a purging stream of an inert gas under conditions which desorb the halogenated hydrocarbon into the purging gas stream.

The invention also provides a canister for use in carrying out the process, for adsorbing halogenated hydrocarbons from a gas stream passed through said canister, said canister having a peripheral side wall, a first end wall with an inlet port and a second end wall with an outlet port, a first filter spaced from said first end wall and closing off a first canister end, a second filter spaced from said second end wall and closing off a second canister end, hydrophobic molecular sieve granular adsorbent material being packed in said canister between said first and second filters, the said adsorbent material consisting essentially of a high silica zeolite material having a ratio of SiO₂/Al₂O₃ in the range of 12 to 50, said molecular sieve adsorbents having pore diameters large enough to permit molecules of the halogenated hydrocarbons to pass therethrough and be selectively adsorbed in the large internal cavities of the crystal framework, whereby the halogenated hydrocarbons are selectively removed from the gas stream, characterised in that the said first and second filters are constituted by fine mesh screens having a mesh size to retain said granular material in said canister, and that resilient means are provided for urging one of the said screens towards the other to compress the granular material between the screens.

According to another aspect of the invention, an anesthetic machine is provided having an inlet port of the aforesaid canister connected to an exhaust port of the machine thereby passing patient exhalent from the anesthetic machine to the canister to absorb anesthetic gases.

According to another aspect of the invention, an apparatus is provided for regenerating the aforesaid canister of adsorbent as connected to an anesthetic machine comprising means for connecting an incoming line of nitrogen gas to the inlet. A means is provided to heat the canister and optionally the nitrogen gas in the incoming line to a temperature in the range of 30°C to 150°C. Means is provided for connecting an outgoing line to the canister outlet and for measuring temperature of nitrogen gas enriched with the desorbed anesthetic in the outgoing line. Regeneration is ceased shortly after the temperature of the nitrogen gas in the outgoing line is at a level of the temperature of the nitrogen gas in the incoming line.

### BRIEF DESCRIPTION OF THE DRAWINGS

Preferred embodiments of the invention are shown in the drawings wherein:
Figure 1 is a schematic of an anesthetic machine with canister connected thereto for removing anesthetics from the patient exhalent;
Figure 2 is a section through the canister of Figure 1;
Figure 3 is a schematic of the apparatus used to regenerate the adsorbent material in the canister of Figure 2;
Figure 4 is a schematic of the multi-stage fractional distillation system for separating components of the anesthetic adsorbed by the canister coupled to the anesthetic machine.
Figure 5 is a plot of the inlet and outlet concentrations versus time for an airstream saturated with isoflurane passed into a canister of adsorbent material.
Figure 6 is a plot of the net amounts of isoflurane evaporated, exhausted and retained in the canister versus time.
Figure 7 is a plot of the concentration versus time of concentration of isoflurane in the purging gas stream exiting from the recovery system and;
Figure 8 is a plot versus time of the net volume of isoflurane lost in the regenerative gas stream exiting the recovery system.

### DETAILED DESCRIPTION OF THE PREFERRED EMBODIMENTS

According to the invention, a system is provided which can recover a variety of halogenated hydrocarbons and purify the recovered compounds. Typical halogenated hydrocarbons include bromo-, chloro- and/or fluoroethers, fluorinated alkyl ethers, chlorofluorohydrocarbons, chlorofluoroethers and their derivatives. Anesthetic gases are well known types of halogenated hydrocarbons which include isoflurane, enflurane, halthane, and methoxyflurane. Other well known halogenated hydrocarbons include the variety of Freons (trade mark) such as trichlorofluromethane, and dichlorodifluoromethane. This family of halogenated hydrocarbon compounds which include, for example, an alkyl group or ether group substituted with one or more of chloro, fluoro and bromo groups are readily adsorbed on the high silica zeolite adsorbent and can be readily desorbed from the adsorbent. A preferred aspect of the invention is described with respect to the recovery of various anesthetic gases. It is appreciated that the principles of the invention which are demonstrated by the following embodiments are equally applicable to the recovery of other types of halogenated hydrocarbons.

A variety of organic based anesthetics are used in patient surgery. Common forms of anesthetics are those sold under the trade marks ETHRANE and FORANE by (ANAQUEST of Quebec, Canada). The respective chemical formulae for these anesthetics are as follows:
1,1,2-trifluoro-2-chloroethyl difluoromethyl ether and 1-chloro-2,2,2-trifluoroethyl difluoromethyl ether. Other types of anesthetics are, for example, Halothane (trade mark) of the formula bromochlorotrifluoroethane and Penthrane (trade mark) of the formula 2,2-dichloro-1,1-difluoroethyl methyl ether which are readily available from various suppliers, such as, Hoechst, Ayerst, Abbott, etc.

By way of an anesthetic machine, these anesthetics either singularly or in combination are delivered to the patient in combination with oxygen, nitrous oxide and/or air. As the patient breathes the gas stream containing the anesthetic, a desired degree of unconsciousness is achieved and monitored by an anesthetist. Not all of the anesthetic inhaled by the patient is absorbed into the blood system. In fact, very little of the anesthetic is absorbed. During procedures, the gas flow rate to the patient may be in the range of 0.5 to 7 liters per minute, where the concentration by volume of the anesthetic may be in the range of 0.3% to 2.5%. Normally, the patient exhalent is not recycled via the anesthetic machine. Instead, it is exhausted to the atmosphere by way of appropriate ducting. It is very important to ensure that the patient exhalent is not exhausted into the operating theatre, because the presence of the anesthetics can have a long term effect on the people in the operating room. It is appreciated that the use of the term patient is in a general sense. It is understood that anesthesia is practiced on a variety of mammals not only including humans but also animals such as horses, cattle and other forms of livestock, domestic pets and the like.

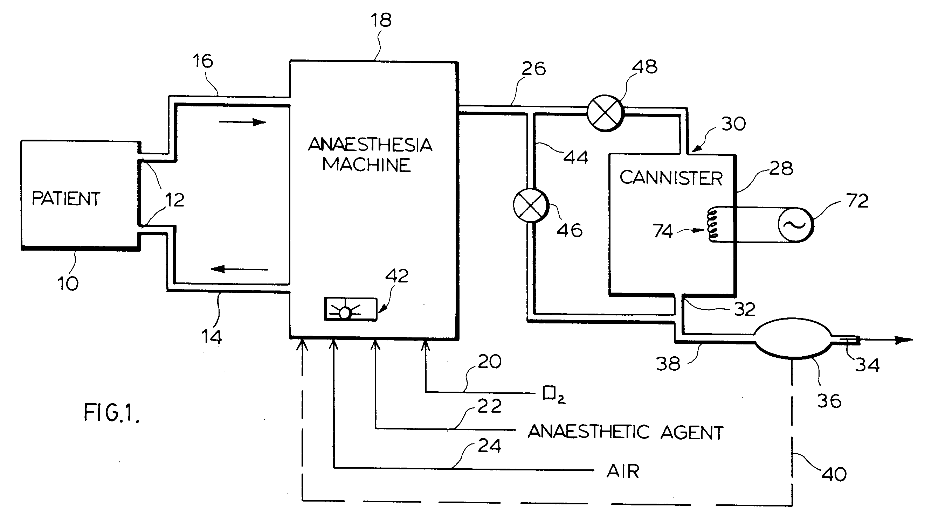

As shown in Figure 1, the patient represented at 10 is connected to a mask 12 having a gas line 14 communicating therewith. The desired mixture of anesthetic gas is delivered in line 14 to the patient 10. The patient exhalent is delivered in line 16 to the anesthetic machine 18. The anesthetic machine 18, which is supplied with oxygen, a source of anesthetic and air in lines 20, 22 and 24, is operated to introduce the desired mixture in line 14. The patient exhalent in line 16 is discharged via line 26. Normally line 26 leads to external ducting for exhausting the anesthetics to atmosphere. In accordance with this invention, a canister 28 having an inlet 30 and an outlet 32 is interposed in line 26 at a position sufficiently downstream of the machine so as to have no or minimal effect on its operation. The patient exhalent in line 26, therefore, flows through the canister 28 before exhausting to atmosphere at 34. The canister 28 is charged with a hydrophobic molecular sieve granular material of silicalite which adsorbs from the patient exhalent stream the organic gaseous anesthetic. Hence, the stream discharge at 34 is free of the anesthetic gases.

An anesthetic sensor 36 may be provided in the exhaust line 38 to sense the presence of anesthetics exiting from the canister 28. It is appreciated that the adsorption front of the adsorbed anesthetics in the bed of adsorbent travels along the bed towards the canister outlet. Such adsorption front will usually have a curved profile across the canister as it approaches the outlet. The curved profile normally assumes an elongated "S" shape. The sensor will sense when any portion of that front has broken through the adsorbent into the outlet. Replacement of the canister is normally required at this time through the bed of adsorbent is not entirely saturated with organic anesthetic. The sensor 36 may be connected via signal line 40 to the anesthetic machine 18. The anesthetic machine may be equipped with a light and/or audible alarm 42 which is actuated when the sensor 36 senses anesthetic gases in line 38. This indicates to the anesthetist that the canister 28 should be replaced so that continued recovery of anesthetics is achieved. It is appreciated that a bypass 44 controlled by valve 46 may be provided to route the patient exhalent past the canister 28 during replacement thereof. In this instance, a valve 48 is provided in line 26 to shut off the supply to canister 28 during replacement of the canister.

The canister is charged with a hydrophobic molecular sieve adsorbent material consisting essentially of a high silica zeolite material having a ratio of SiO₂/Al₂O₃ in the range of 12 to 50, the material having pore diameters large enough to permit molecules of the halogenated hydrocarbons to pass through them and to be selectively adsorbed in the large internal cavities of the crystal framework.

It has been found that the silica polymorphs known as silicalite, F-silicalite and TEA-silicalite are particularly suitable for use in the present invention and are thus preferred, though not, strictly speaking, zeolites, because of a lack of ion-exchange capacity, these molecular sieve materials are included within the terms zeolite or zeolitic molecular sieve as used herein.

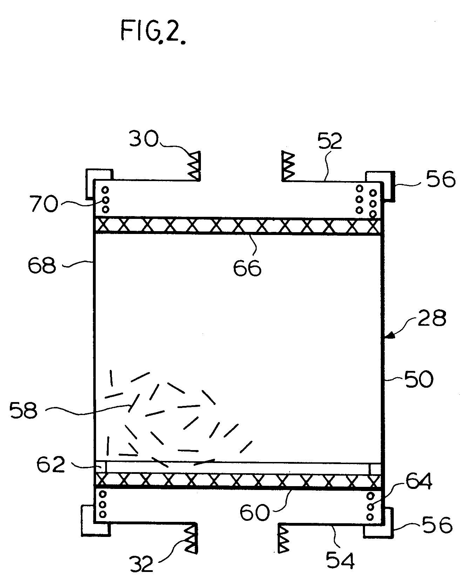

As shown in Figure 2, the canister 28, which may be cylindrical in shape, has a side wall 50 with a first end 52 having an inlet 30. A second end 54 has the outlet 32. It is appreciated that the canister 28 may be disassembled by having releasable fasteners 56 about the perimeter of the side wall 28 to clip respectively the first and second end walls 52 and 54 to the side wall 50. Within the canister 28, the hydrophobic molecular sieve granular material of silicalite 58 is contained. At the second end of the canister, a fine mesh screen 60 is positioned to close off the second end defined by flange 62. The fine mesh screen 60 conforms to the interior shape of the canister side wall 50 which, in this instance, is circular and abuts the flange 62. A coiled spring 64, as spaced between the wall 54 and the fine mesh screen 60, holds the screen in place against the flange 62. With the other end 52 and the fine mesh screen 66 removed, the silicalite material 58 may be charged into the canister 28. Once the silicalite material has achieved a level indicated by arrow 68, the screen 66 is placed in the canister. A spring 70 is positioned between the wall 52 and the screen 66. When the clips 56 are clamped in position, the spring pushes the fine mesh screen 66 against the silicalite material 58 to compress and hold the silicalite material in place in the canister 28. This ensures that the silicalite material remains relatively fixed in the canister during use.

The patient exhalent in line 26 from the anesthetic machine 18 of Figure 1 is naturally moist. This has presented significant problems in the past in attempting to recover organic anesthetics from the moist patient exhalent. It has been discovered that the use of a hydrophobic molecular sieve granular material of silicalite overcomes those problems. The silicalite material has a pore diameter which permits the material to selectively adsorb and remove the organic gaseous anesthetic from the humid patient exhalent and which minimizes coadsorption of water molecules in the patient exhalent. The benefits in using silicalite adsorbents is that there is no bacterial growth on the adsorbents which can become a problem because of the presence of bacteria in the patient exhalent. The adsorbent is non-flammable in the presence of oxygen. This is a significant drawback with organic forms of adsorbents since for certain concentrations of oxygen, the organic adsorbents are at least flammable if not explosive. The silicalite adsorbent is inert so that minimal if any decomposition of the anesthetic agent is induced whereas with organic adsorbents, such as activated carbon, hydrochloric acid can be produced in the presence of iron by way of decomposition of the halogenated anesthetics. The inert silicalite adsorbents are readily re-sterilized using ozone, steam, peroxide or other disinfectants without in any way affecting the adsorptive reuse characteristics of the adsorbent. The silicalite adsorbents are found to be microwave transparent. Therefore, regeneration can be accomplished using microwave heating.

A preferred form of silicalite is that manufactured and sold by Union Carbide under the trade mark "S-115 Silicalite". The chemical properties of S-115 Silicalite are as follows:
Chemical properties (greater than) 99% SiO₂ (less than) 1% aluminum oxide.
The Silicalite has the following physical properties:

| Free aperture | |
|---|---|
| Zig-zag channels | 0,54 nm (5.4 Å) |
| Straight channels | 0,575 x 0,515 nm (5.75 x 5.15 Å) |
| Pore volume | 0.19 cc/gm |
| Pore size | approx. 0,6 nm (6 ångström) in diameter |
| Crystal density | 1.76 gm/cc |
| Largest molecule adsorbed | Benzene |
| Form | Powder, Bonded Bead or Pellet |

By use of a silicalite material having those properties, it has been discovered that the organic anesthetics are adsorbed by the silicalite while other components of the patient exhalent, including moisture, pass through. Hence, a minimum of moisture is retained in the canister. Supplemental heating of the canister 28, as shown in Figure 1, may be provided by control 72 for heater 74. The purpose of the heat is to ensure that the canister 28, during use on the anesthetic machine, remains at a temperature which prevents the moisture in the patient exhalent condensing on the silicalite material in the canister and also on the canister surfaces.

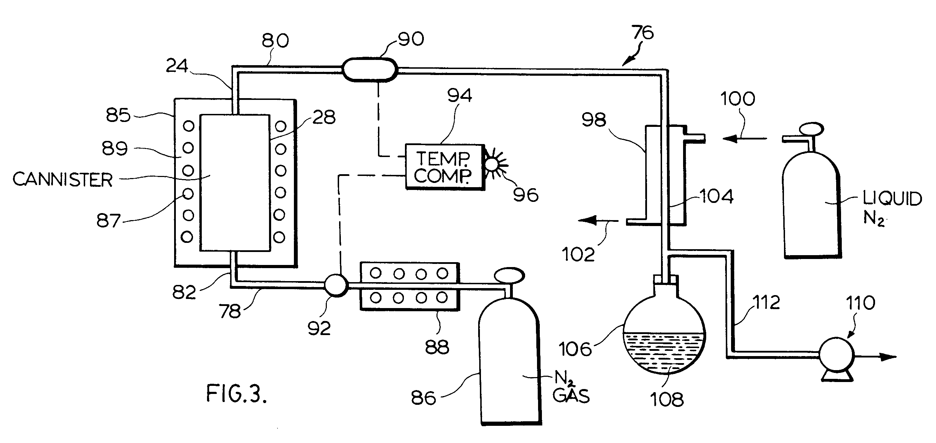

Once it has been determined that the silicalite material in the canister is saturated with adsorbed organic anesthetic, or that the adsorption front has broken through to the outlet, the canister has to be replaced in the manner discussed. To regenerate the silicalite material in the canister 28 and to recover the anesthetic components for reuse, a silicalite regeneration system 76 is shown in Figure 3. The system permits interposing canister 28 in lines 78 and 80 by couplings 82 and 84 which connect to the inlet and outlet 30 and 32 of the canister 28. The canister may be optionally heated within a conventional oven 85. An inert purging gas is passed through the silicalite material of the canister 28 to desorb the organic anesthetics from the silicalite granular material. In accordance with a preferred aspect of this invention, nitrogen gas or air is used as the purging gas. To enhance the desorption of the adsorbed organic anesthetics, the silicalite material is preferably heated to a temperature range of 30°C to 150°C. It is appreciated that with other types of halogenated hydrocarbons, different temperature ranges may be necessary to effect desorption of the compounds.

In order to heat the silicalite material within the canister to this temperature the oven 85 having heating coils 87 surrounded by insulating material 89 is controlled on the basis of prior experimentation in a manner to ensure that the silicalite is in this temperature range for passing of the purging gas through the canister. It is understood that in view of the transparency of the silicalite adsorbent to microwaves, then a microwave oven may be substituted for the conventional oven 85.

The silicalite material in canister 28 during regeneration may either be heated by direct application of heat to the canister or by heating the nitrogen gas or air purging stream. In accordance with the embodiment shown in Figure 3, the nitrogen gas from the source 86 may also be heated in heater 88 to a desired temperature in the range of 30° to 150°C. The purging gas passes through the silicalite material of the canister 28 where the fine mesh screen, as shown in Figure 2, serve to retain the silicalite material in the canister. Hence any desired flow rate of purging gas may be used. The purging gas exits the canister 28 through line 80 and passes through a temperature sensor 90. Temperature sensor 90 provides an indication of the temperature of the purging gas in line 80. When the temperature of the purging gas in the exit line achieves a temperature nearing that of the temperature in the entrance lines 78, it has been determined that the silicalite material is at a temperature approximating the inlet temperature and that most of the organic anesthetic is desorbed. The system is then run for a desired period of time beyond that point to complete desorption. That aspect of the process may be automated and a temperature sensor 92 may be included in the inlet side to measure the temperature of the incoming stream. By way of suitable microprocessor, the signals from temperature sensors 90 and 92 may be fed to a control system 94 which compares the temperatures and actuates a signal 96 to indicate that canister regeneration is complete. It is appreciated, that regeneration of the silicalite adsorbent may take place at lower temperatures outside of the preferred range. For example, regeneration of absorbent can be achieved at temperatures as low as 25°C where the time for regeneration is thereby extended.

It is appreciated that in the alternative, silicalite adsorbent carrying anesthetic compounds may be removed from the canister and placed with adsorbent removed from other canisters. The collected adsorbent may then be regenerated in a separate vessel in a manner as discussed with respect to a single canister.

The purging gas continues in line 80 through condenser 98. The purpose of the condenser is to remove, in liquid form, the organic anesthetics from the purging gas. Liquid nitrogen at a cryogenic temperature is fed through the condenser 98 via its inlet 100 and exit 102. This provides sufficiently cool temperatures in the line 104 of the condenser to cause the organic anesthetics to condense and permits collection in vessel 106 of liquid form anesthetics 108. To assist in the condensing of the organic anesthetics, a partial vacuum is drawn in line 104 by vacuum pump 110 connected to line 104 via line 112. The condensed liquid 108 then consists primarily of the organic anesthetics. In the course of one day, several operations may be conducted involving the same anesthetic machine 18. It may require many operations to saturate the canister 28 with anesthetics from the patient exhalent. During the different operations, it is appreciated that different anesthetics may be used. For example, Forane (trade mark) or Ethrane (trade mark) may be used separately or in combination with or without Halothane (trade mark). When the canister is saturated, two or more gases may be present inside. Hence, liquid 108 will correspondingly consist of a mixture of anesthetic components.

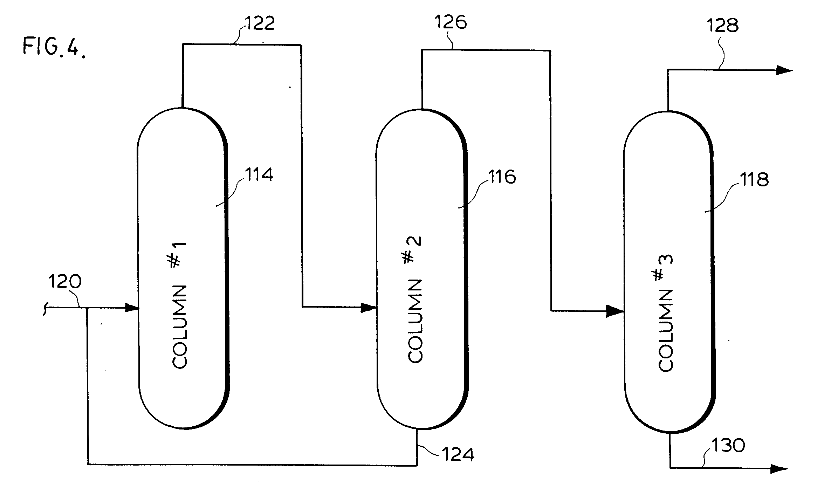

Regardless of the composition of the liquid 108, it is important to purify it before reuse. In accordance with standard practice, anesthetics must have a high purity level normally in excess of 90% providing remaining impurities are non-toxic. To achieve that purity, the liquid 108 is subjected to fractional distillation. A preferred system is shown in Figure 4 consisting of a multi-stage fractional distillation comprising three columns 114, 116 and 118. The liquid 108 is fed to column 114 via line 120. Sufficient heat is applied to the bottom of column 114 to cause the liquid 108 to boil and provide a vapor take-off in line 122. The vapor 122 is fed to column 116 where heat is applied to cause boiling of the vapor 122 as it condenses in column 116. The bottoms of columns 116 are removed via line 124 for recycle with new product into column 114. The vapors removed from column 116 via line 126 are fed to column 118. The vapors in line 126 condense in column 118 and with heat supplied thereto, cause boiling resulting in a take-off of two fractions, one in vapor phase in line 128 and secondly in liquid phase in line 130. Assuming that two anesthetics are in the liquid 108, the system of Figure 4 separates them to provide desired purities in the lines 128 and 130. For example, with Forane and Ethrane, there is a difference in boiling points of approximately 8°C which is sufficient to provide separation of the Ethrane from the Forane.

Bacteria is present in the patient exhalent. It has been found, however, that a bacteria in the patient exhalent is not adsorbed on the silicalite material to any appreciable extent. Hence, the anesthetic produced by fractional distillation and particularly as provided in lines 128 and 130 is not contaminated and is ready for reuse. In accordance with this invention, an inexpensive process and apparatus is provided for what in essence is the manufacture of anesthetic gases from mixtures which are normally discharged to the atmosphere. Significant economic advantages are realized.

Without limiting the scope of the appended claims, the following examples exemplify preferred aspects of the inventive process.

### EXAMPLE 1

A canister of the type shown in Figure 2 was subjected to a known flow rate of air with a known concentration of the anesthetic isoflurane while monitoring the inlet and outlet concentration of isoflurane in the canister outlet until saturation of the adsorbent in the canister was detected by breakthrough of the adsorption front. The apparatus was set up to generate a constant concentration of isoflurane in the air stream. The source of air was from a cylinder of "Zero" grade air a portion of the air metered through a flow meter was passed through two midget impingers each containing 15ml of the anesthetic isoflurane. A third impinger prevented droplets of the isoflurane from being carried over and into the air stream. The isoflurane saturated air was then mixed with the zero air. The total flowrate was measured with a second flow meter. A dry gas meter was installed at the canister outlet to provide confirmation of the flow rate indicated by the upstream flow meter. The outlets and inlets were sampled periodically throughout the tests by way of a Miran (trade mark) 1A infrared analyzer. This instrument is a variable wavelength, variable pathlink analyzer capable of measuring isoflurane to concentrations well below 1 ppm. The instrument was calibrated before use to provide accurate readouts of the inlet and outlet concentrations of the canister.

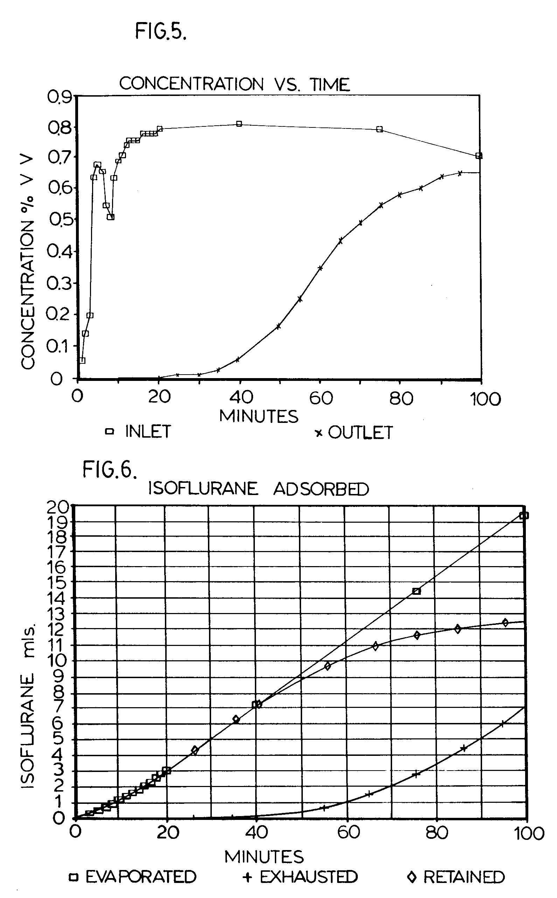

Figure 5 is a plot of the inlet and outlet concentrations versus time at the canister. The inlet concentration was about .77% by volume for most of the program and the average flow rate was approximately 5.2 meters per minute. Breakthrough started to occur after about 30 minutes. The canister appeared to be fully saturated after about 100 minutes. At that point the outlet value for isoflurane concentration was only slightly less than the inlet value. The inlet value dropped because most of the isoflurane had been evaporated. There were 8ml of isoflurane remaining in the impingers at the end program.

Figure 6 is a plot of the net amounts of isoflurane evaporated, exhausted and retained versus time as calculated from the measured flow of isoflurane concentrations. The figure shows that about 19.5 ml were evaporated and about 12.7 ml were expected to have been adsorbed by the adsorbent in the canister at the end of the test run.

The canister of adsorbent was regenerated by use of an apparatus of the type shown in Figure 3. The canister was heated in an oven to a temperature of approximately 140°C. The nitrogen gas passed through the canister was at a flow rate of approximately 1.3 litres per minute during regeneration. During such regeneration the nitrogen gas emerging from the coal trap was monitored for isoflurane.

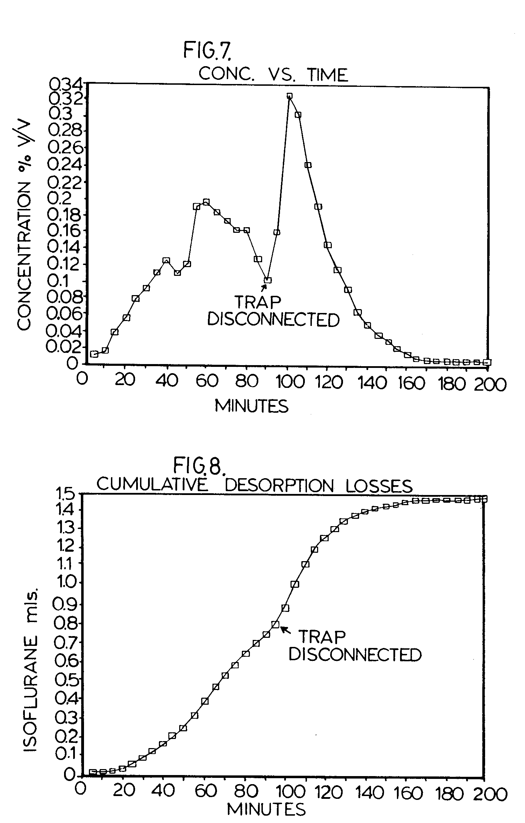

Figure 7 is a plot of the concentration versus time for the monitored isoflurane concentration in the emerging nitrogen gas stream.

Figure 8 is a plot of the net volume of isoflurane lost versus time based on a flow rate of the 1.3 litres per minute of the regeneration gas.

The volume of isoflurane recovered from the flow trap was 11 ml. The amount expected was 12.7 ml. - 1.5 ml. = 11.2 mls. No water was recovered as expected since dry air was used. Furthermore, the adsorbent is principally hydrophobic. The results of the tests are therefore summarized in the following Table 1.

**TABLE 1**

| Laboratory Test Results - Summary | |
|---|---|
| Average inlet concentration | 0.76% |
| Amount of Isoflurane in impinger | 30.0 mls |
| Amount remaining | 8.0 mls |
| Calculated isoflurane entering canister | 19.5 mls |
| Isoflurane exhausted | 6.7 mls |
| Amount of isoflurane expected | 12.7 mls |
| Amount lost during desorption | 1.5 mls |
| Net amount expected from recovery | 11.2 mls |
| Actual amount recovered | 11.0 mls |

Approximately 90% of the isoflurane was recovered by thermal desorption using a low purge flow rate for the purging gas. According to this particular set up the canister capacity for isoflurane is approximately 13 mls or 18 grams of the isoflurane. The volume of adsorptive material in the canister was approximately 185 grams of the SR-115 silica zeolite adsorbent material.

### Example 2

The procedure of Example 1 was repeated with a view to establishing what the effect of the presence of water vapour in the gas stream had on the adsorption of the anesthetic gases. An impinger, containing water, was used to add moisture to the gas stream carrying the anesthetic gases. The average absolute humidity of 2.2% v/v was established. The inlet concentration of isoflurane was 0.84% by volume and the average flowrate was 5.2 litres per minute. Breakthrough occurred in approximately 25 minutes and the canister was completely saturated after approximately 78 minutes. Approximately 12.1 mls of isoflurane was adsorbed in the canister which is similar to the amount adsorbed in Example 1 under similar flowrate conditions. Hence the presence of moisture did not appreciably affect the adsorption of isoflurane.

The procedure of Example 1 was followed to desorb the isoflurane from the canister. Similar volume of isoflurane was recovered along with a minimal volume of water. Fractional distillation was used to separate the isoflurane from the water.

### Example 3

As the canister approaches saturation with adsorbed isoflurane continued passage of the gas stream through the canister has the potential for stripping isoflurane from the canister. The following procedure was established to determine if stripping could occur. A canister with 185 grams of silicalite was saturated with isoflurane. Air was then passed through the canister at a rate of about 6 litres per minute. The air at the exit of the canister was monitored for isoflurane using the Miran (trade mark) analyzer. At the beginning of the passage of the air stream, approximately 1.5 ml of isoflurane was removed from the saturated canister. Thereafter there was a nearly constant but extremely low concentration of isoflurane detected at the exit of the canister. This low concentration could not be accurately measured but was estimated to be at about .01 to .02% v/v for approximately .2 ml of liquid isoflurane per hour. Stripping of isoflurane from saturated or partially saturated canisters is therefore avoided and does not have a significant impact on the net amount of isoflurane that can be recovered from a gas stream.

### Example 4

Several canisters were used in a "real" situation by coupling the individual canisters to anesthetic machines which were in use at the Toronto General Hospital. Recovery of isoflurane from these canisters by thermal desorption in accordance with the procedure of Example 1 revealed that certain impurities were appearing in the recovered mixture prior to the purification step. To determine the extent of impurities the following procedure was followed.

A new canister was loaded with 185 grams silicalite and regenerated at 120 degrees centigrade before use. The clean canister was coupled to a new anesthetic machine which was then put into use. After saturation of the canister it was then subjected to the procedure of Example 1 for recovery of the isoflurane. Recovery was carried out a desorption temperature of 120 degrees centigrade. The impurities identified in the recovered mixture were as follows:
1. 1-1-1-trifluoro-2-chloroethane;
2. bromochloro-1-1-difluoroethylene;
3. ethanal;
4. ethylene oxide;
5. trichlorofluoromethane;
6. dichlorodifluoromethane;
7. isopropyl alcohol;
8. 2-2-2 trifluoroethanol.
The fact that the above impurities appeared as desorbed from the adsorbent indicates that the high silica zeolite, adsorbent is capable of absorbing a variety of halogenated hydrocarbons and in turn desorbing such compounds at suitable desorption temperatures. It is thought that impurity # 8 is the result of the degradation of the isoflurane. Impurity # 2 is thought to be a breakdown product of halothane, ethanol (acetaldehyde) is possibly present as a patient exhalent, ethylene oxide and isopropyl alcohol are common chemicals used as disinfectants in the hospital. Impurities 5 and 6 are commonly known as Freon 11 (trade mark) and Freon 12 (trade mark). It is believed these compounds were present in the new anesthetic machine as potential filler gases, however, the presence of such gases indicate that these types of halogenated hydrocarbons are adsorbed onto the adsorbent of the canister and can be subsequently desorbed by temperature desorption.

Although the use of this canister has been demonstrated in association with an anesthetic machine, it is appreciated that the canister may be used in other systems to adsorb other types of halogenated hydrocarbons such as those commonly used as solvents, blowing agents, refrigerants, aerosol propellants and the like. Suitable systems may be set up to collect the vapours of these various agents and direct them through canisters which function in the same manner as the canisters specifically exemplified. Canisters can then be subjected to temperature desorption to provide for recovery and subsequent purification of the adsorbed halogenated hydrocarbons.

## Claims

1. A process for the recovery of at least one halogenated hydrocarbon from a gas stream which also includes water vapours, comprising the steps of passing the gas stream through a bed of an adsorbent material to adsorb the halogenated hydrocarbon into the bed with minimal adsorption of water vapour, and removing the bed of adsorbent material containing the adsorbed halogenated hydrocarbon from the gas stream;
characterised in that the adsorbent material consists essentially of a high silica zeolite material having a ratio of SiO₂/Al₂O₃ in the range of 12 to 50, the bed having pore diameters large enough to permit molecules of the halogenated hydrocarbon to pass therethrough for selective adsorption in the larger internal cavities of the crystal framework; and that said bed of absorbent material is regenerated by exposing it to a purging stream of an inert gas under conditions which desorb the halogenated hydrocarbon into the purging gas stream.

2. A process according to claim 1 wherein the high silica zeolite material is selected from the group consisting of silicalite, F-silicalite and TEA-silicalite.

3. A process according to claim 1, wherein said halogenated hydrocarbons are removed from said purging gas by condensing said halogenated hydrocarbons out of said purging gas in a liquid form.

4. A process according to claim 1, 2 or 3, wherein the halogenated hydrocarbon is bromochlorofluoro ether, fluorinated alkyl ether, chlorofluorocarbon or chlorofluoro ether, or their derivatives.

5. A process according to claim 4 wherein said halogenated hydrocarbons are selected from fluorochloro ethers and bromochlorofluoro hydrocarbon anesthetic gases.

6. A process according to any proceeding claim, wherein said moist gas stream is patient exhalent exhausted from an anesthetic machine, said patient exhalent including anesthetic gases which are adsorbed on said absorbent material, said anesthetic gases being desorbed from said absorbent and purified for re-use as anesthetic on a patient.

7. A process according to claim 8, wherein said bed of adsorbent material is contained in a canister having an inlet and an outlet connected to an exhaust for patient exhalent from said anesthetic machine, the said canister being replaced by another like canister when breakthrough occurs, and comprising the steps of sensing said patient exhalent exiting from said canister outlet for presence in said patient exhalent of said breakthrough and actuating a detectable alarm to indicate that said bed of silicalite material is saturated and requires replacement.

8. A process according to claim 7 wherein said saturated bed of adsorbent material is regenerated in said canister, and said purging gas is passed through said canister via said inlet and outlet to desorb said organic anesthetic from said silicalite material.

9. A process according to claim 8, wherein said canister is heated to elevate temperature of said adsorbent material during regeneration to assist in desorption of said organic anesthetic.

10. A process according to any proceeding claim, wherein said purging gas is nitrogen or air.

11. A process according to claim 7, wherein said patient exhalent as derived from one or more patients includes at least two different organic anesthetics, said adsorbent material adsorbing both of the said different organic anesthetics which are desorbed by said purging gas and condensed therefrom in an isolated liquid form, said isolated liquid being purified by fractional distillation of said isolated liquid to separate the respective organic anesthetics into individual organic anesthetics.

12. A process according to claim 11, wherein two different anesthetics are recovered by fractional distillation, said anesthetics being 1,1,2-trifluoro-2-chloroethyl difluoromethyl ether and 1-chloro-2,2,2-trifluoroethyl difluoromethyl ether.

13. A process according to claim 11 or 12, wherein said saturated adsorbent material is heated to temperatures in the range of 30°C to 150°C, said inert purging gas is nitrogen or air.

14. A process according to claim 11, 12 or 13, wherein said purging gas stream with desorbed anesthetic is passed through a condenser operating at a sufficiently low temperature to condense out of said purging gas stream said anesthetics in liquid form.

15. A process according to claim 14, wherein said condensed liquid is subjected to multiple-stage fractional distillation to purify said anesthetics.

16. A process according to claim 15, wherein said purging gas is heated to said temperature in the range of 30°C to 150°C, said silicalite material being heated by nitrogen purging gas during desorption of said anesthetics.

17. A process according to claim 16, wherein said temperature range to which said adsorbent and gas are heated is 30°C to 150°C.

18. A canister for use in the process of any one of claims 1 to 17, for adsorbing halogenated hydrocarbons from a gas stream passed through said canister, said canister having a peripheral side wall, a first end wall with an inlet port and a second end wall with an outlet port, a first filter spaced from said first end wall and closing off a first canister end, a second filter spaced from said second end wall and closing off a second canister end, hydrophobic molecular sieve granular adsorbent material being packed in said canister between said first and second filters, the said adsorbent material consisting essentially of a high silica zeolite material having a ratio of SiO₂/Al₂O₃ in the range of 12 to 50, said molecular sieve adsorbents having pore diameters large enough to permit molecules of the halogenated hydrocarbons to pass therethrough and be selectively adsorbed in the large internal cavities of the crystal framework, whereby the halogenated hydrocarbons are selectively removed from the gas stream, characterised in that the said first and second filters are constituted by fine mesh screens having a mesh size to retain said granular material in said canister, and that resilient means are provided for urging one of the said screens towards the other to compress the granular material between the screens.

19. A canister according to claim 18, wherein said adsorbent material has a pore size of approximately 0,6 nm (6 ångström).

20. A canister according to claim 18 or 19, wherein said canister walls are formed of a corrosion resistant metal.

21. An anesthetic machine in which the said inlet port of a canister according to claim 18, 19 or 20 is connected to an exhaust port of the anesthetic machine to pass thereby patient exhalent from said anesthetic machine through said canister and thereby absorb anesthetic gases from the patient exhalent.

22. An apparatus for regenerating the canister of silicalite of claim 18, 19 or 20, comprising means for connecting an incoming line of nitrogen gas or air to said inlet, means for heating said nitrogen gas or air in said incoming line to a temperature in the range of 30°C to 150°C, means for connecting an outgoing line to said outlet and means for measuring temperature in said outgoing line to said nitrogen gas enriched with desorbed anesthetic.

23. An apparatus according to claim 22, wherein said outgoing line is connected to a condenser operating at a sufficiently low temperature to remove in liquid form said anesthetics from said nitrogen gas stream, and including means for collecting said liquid condensate from said condenser.

## Patentansprüche

1. Verfahren für die Wiedergewinnung mindestens eines halogenierten Kohlenwasserstoffes aus einem auch Wasserdämpfe aufweisenden Gasstrom, bei dem der Gasstrom durch ein Bett eines Adsorptionsmittels geleitet wird, um den halogenierten Kohlenwasserstoff in dem Bett bei minimaler Adsorption von Wasserdampf zu adsorbieren, und bei dem das Bett aus Adsorptionsmittel, welches den aus dem Gasstrom adsorbierten halogenierten Kohlenwasserstoff enthält, beseitigt wird;
dadurch gekennzeichnet, daß das Adsorptionsmittel im wesentlichen aus einem siliziumdioxidreichen Zeolitmaterial mit einem SiO₂/Al₂O₃-Verhältnis im Bereich von 12 bis 50 besteht, das Bett Porendurchmesser hat, die ausreichend groß sind, um Moleküle des halogenierten Kohlenwasserstoffes für eine selektive Adsorption in den größeren inneren Hohlräumen des Kristallgerüsts durchzulassen; und daß das Adsorptionsmittelbett regeneriert wird, indem es einem Inertgas-Spülstrom unter Bedingungen ausgesetzt wird, bei denen der halogenierte Kohlenwasserstoff in den Spülgasstrom desorbiert wird.

2. Verfahren nach Anspruch 1, bei dem das siliziumdioxidreiche Zeolitmaterial aus der aus Silicalit, F-Silicalit und TEA-Silicalit bestehenden Gruppe ausgewählt wird.

3. Verfahren nach Anspruch 1, bei dem die halogenierten Kohlenwasserstoffe aus dem Spülgas entfernt werden, indem die halogenierten Kohlenwasserstoffe aus dem Spülgas in flüssiger Form kondensiert werden.

4. Verfahren nach Anspruch 1, 2 oder 3, bei dem es sich bei dem halogenierten Kohlenwasserstoff um Bromchlorfluorether, fluorierten Alcylether, Chlorfluorkohlenstoff oder Chlorfluorether oder deren Derivate handelt.

5. Verfahren nach Anspruch 4, bei dem die halogenierten Kohlenwasserstoffe aus der aus Fluorchlorethern und Bromchlorfluorkohlenwasserstoff-Narkosegasen bestehenden Gruppe ausgewählt werden.

6. Verfahren nach einem der vorhergehenden Ansprüche, bei dem es sich bei dem feuchten Gasstrom um von einer Narkosemaschine abgegebene Patienten-Ausatmungsluft handelt, die Narkosegase aufweist, welche von dem Adsorptionsmittel adsorbiert werden und welche von dem Adsorptionsmittel zwecks Wiederverwendung als Narkosemittel für einen Patienten desorbiert und gereinigt werden.

7. Verfahren nach Anspruch 6, bei dem das Adsorptionsmittelbett in einem Kanister untergebracht ist, der einen mit einem Auslaß für Patienten-Ausatmungsluft von der Narkosemaschine verbundenen Einlaß und einen Auslaß aufweist und der gegen einen anderen gleichartigen Kanister ausgetauscht wird, wenn ein Durchbruch erfolgt, wobei der Durchbruch in der aus dem Auslaß des Kanisters austretenden Patienten-Ausatmungsluft erfaßt wird und ein wahrnehmbarer Alarm ausgelöst wird, um anzuzeigen, daß das Bett aus Silicalitmaterial gesättigt ist und ausgetauscht werden muß.

8. Verfahren nach Anspruch 7, bei dem das gesättigte Adsorptionsmittelbett in dem Kanister regeneriert wird und das Spülgas durch den Kanister über den Einlaß und den Auslaß geleitet wird, um das organische Narkosemittel von dem Silicalitmaterial zu desorbieren.

9. Verfahren nach Anspruch 8, bei dem der Kanister erhitzt wird, um die Temperatur des Adsorptionsmittels während der Regenerierung zu steigern und dadurch die Desorption des organischen Narkosemittels zu unterstützen.

10. Verfahren nach einem der vorhegehenden Ansprüche, bei dem das Spülgas Stickstoff oder Luft ist.

11. Verfahren nach Anspruch 7, bei dem die von einem oder mehreren Patienten kommende Patienten-Ausatmungsluft mindestens zwei unterschiedliche organische Narkosemittel aufweist, das Adsorptionsmittel die beiden unterschiedlichen organischen Narkosemittel adsorbiert, die mittels des Spülgases desorbiert und in Form einer isolierten Flüssigkeit aus dem Spülgas heraus kondensiert werden, und die isolierte Flüssigkeit durch fraktionierte Destillation der isolierten Flüssigkeit gereinigt wird, um die betreffenden organischen Narkosemittel in einzelne organische Narkosemittel zu trennen.

12. Verfahren nach Anspruch 11, bei dem zwei unterschiedliche Narkosemittel durch fraktionierte Destillation zurückgewonnen werden, wobei es sich bei den Narkosemitteln um 1,1,2-Trifluor-2-chlorehtyldifluormethylether und 1-Chlor-2,2,2-trifluorethyldifluormethylether handelt.

13. Verfahren nach Anspruch 11 oder 12, bei dem das gesättigte Adsorptionsmittel auf Temperaturen im Bereich von 30 °C bis 150 °C erhitzt wird, wobei das inerte Spülgas Stickstoff oder Luft ist.

14. Verfahren nach Anspruch 11, 12 oder 13, bei dem der Spülgasstrom mit desorbiertem Narkosemittel durch einen Kondensator hindurchgeleitet wird, der mit ausreichend niedriger Temperatur betrieben wird, um die Narkosemittel aus dem Spülgasstrom in flüssiger Form auszukondensieren.

15. Verfahren nach Anspruch 14, bei dem die kondensierte Flüssigkeit einer mehrstufigen fraktionierten Destillation unterzogen wird, um die Narkosemittel zu reinigen.

16. Verfahren nach Anspruch 15, bei dem das Spülgas auf die Temperatur im Bereich von 30 °C bis 150 °C erhitzt wird und das Silicalitmaterial durch Stickstoffspülgas während der Desorption der Narkosemittel erhitzt wird.

17. Verfahren nach Anspruch 16, bei dem der Temperaturbereich, auf den das Adsorptionsmittel und das Gas erhitzt werden, 30 °C bis 150 °C beträgt.

18. Kanister zur Verwendung bei dem Verfahren nach einem der Ansprüche 1 bis 17 zum Adsorbieren von halogenierten Kohlenwasserstoffen aus einem durch den Kanister hindurchgeleiteten Gasstrom, wobei der Kanister eine periphere Seitenwand, eine mit einem Einlaß versehene erste Stirnwand und eine mit einem Auslaß versehene zweite Stirnwand, ein in Abstand von der ersten Stirnwand angeordnetes und ein erstes Kanisterende abschließendes erstes Filter und ein in Abstand von der zweiten Stirnwand angeordnetes und ein zweites Kanisterende abschließendes zweites Filter aufweist, wobei in den Kanister zwischen dem ersten und dem zweiten Filter hydrophobes, körniges Molekularsieb-Adsorptionsmittel eingefüllt ist, das im wesentlichen aus einem siliziumdioxidreichen Zeolitmaterial mit einem SiO₂/Al₂O₃-Verhältnis im Bereich von 12 bis 50 besteht, das Porendurchmesser hat, die ausreichend groß sind, um Moleküle der halogenierten Kohlenwasserstoffe für eine selektive Adsorption in den größeren inneren Hohlräumen des Kristallgerüsts durchzulassen, wodurch die halogenierten Kohlenwasserstoffe aus dem Gasstrom selektiv beseitigt werden, dadurch gekennzeichnet, daß das erste und das zweite Filter von feinmaschigen Sieben gebildet sind, die eine solche Maschengröße haben, daß das körnige Material in dem Kanister zurückgehalten wird, und daß eine nachgiebige Anordnung vorgesehen ist, um eines der Siebe in Richtung auf das andere Sieb zu drücken und dadurch das körnige Material zwischen den Sieben zusammenzupressen.

19. Kanister nach Anspruch 18, bei dem das Adsorptionsmittel eine Porengröße von näherungsweise 0,6 nm (6 Angström) hat.

20. Kanister nach Anspruch 18 oder 19, bei dem die Kanisterwände von korrosionsbeständigem Metall gebildet sind.

21. Narkosemaschine, bei welcher der Einlaß eines Kanisters nach Anspruch 18, 19 oder 20 mit einem Auslaß der Narkosemaschine verbunden ist, um Patienten-Ausatmungsluft von der Narkosemaschine durch den Kanister hindurchzuleiten und dadurch aus der Patienten-Ausatmungsluft Narkosegase zu adsorbieren.

22. Vorrichtung zum Regenerieren des Silicalitkanisters nach Anspruch 18, 19 oder 20, mit einer Anordnung zum Verbinden einer Zufuhrleitung für Stickstoffgas oder Luft mit dem Einlaß, einer Anordnung zum Erhitzen des Stickstoffgases oder der Luft in der Zufuhrleitung auf eine Temperatur im Bereich von 30 °C bis 150 °C, eine Anordnung zum Verbinden einer abgehenden Leitung mit dem Auslaß und einer Anordnung zum Messen der Temperatur des mit desorbiertem Narkosemittel angereicherten Stickstoffgases in der abgehenden Leitung.

23. Vorrichtung nach Anspruch 22, wobei die abgehende Leitung mit einem Kondensator verbunden ist, der bei einer hinreichend niedrigen Temperatur betrieben ist, um die Narkosestoffe aus dem Stickstoffgasstrom in flüssiger Form zu beseitigen, und wobei eine Anordnung vorgesehen ist, um das flüssige Kondensat von dem Kondensator zu sammeln.

## Revendications

1. Procédé pour séparer au moins un hydrocarbure halogéné d'un courant gazeux qui comprend également des vapeurs d'eau, comprenant les étapes de passage du courant gazeux à travers un lit d'une matière adsorbante pour adsorber l'hydrocarbure halogéné dans le lit avec une adsorption minimale de vapeur d'eau et d'interruption du contact entre le lit de matière adsorbante contenant l'hydrocarbure halogéné adsorbé et le courant gazeux ;
caractérisé en ce que la matière adsorbante consiste essentiellement en une matière zéolitique à haute teneur en silice ayant un rapport SiO₂/Al₂O₃ compris dans l'intervalle de 12 à 50, le lit ayant des diamètres de pores suffisamment grands pour permettre aux molécules de l'hydrocarbure halogéné de le traverser pour une adsorption sélective dans les plus grandes cavités internes du réseau cristallin ; et ledit lit de matière adsorbante est régénéré par mise en contact de ce lit avec un courant de purge constitué d'un gaz inerte dans des conditions provoquant la désorption de l'hydrocarbure halogéné dans le courant de gaz de purge.

2. Procédé suivant la revendication 1, dans lequel la matière zéolitique à haute teneur en silice est choisie dans le groupe consistant en silicalite, F-silicalite et TEA-silicalite.

3. Procédé suivant la revendication 1, dans lequel les hydrocarbures halogénés sont séparés du gaz de purge par condensation des hydrocarbures halogénés sous forme liquide à partir dudit gaz de purge.

4. Procédé suivant la revendication 1, 2 ou 3, dans lequel l'hydrocarbure halogéné est un bromochlorofluoroéther, un éther alkylique fluoré, un chlorofluorocarbone ou un chlorofluoro-éther, ou bien un de leurs dérivés.

5. Procédé suivant la revendication 4, dans lequel les hydrocarbures halogénés sont choisis entre des fluorochloro-éthers et des gaz anesthésiants consistant en bromochlorofluorohydrocarbures.

6. Procédé suivant l'une quelconque des revendications précédentes, dans lequel le courant gazeux humide est le courant gazeux expiré par un patient, évacué d'un appareil anesthésiant, ledit courant gazeux expiré par le patient comprenant des gaz anesthésiants qui sont adsorbés sur ladite matière adsorbante, lesdits gaz anesthésiants étant désorbés dudit adsorbant et purifiés pour une réutilisation comme agent anesthésiant sur un patient.

7. Procédé suivant la revendication 6, dans lequel le lit de matière adsorbante est logé dans une cartouche ayant un orifice d'admission et un orifice de sortie connectés à un orifice d'évacuation, pour le courant gazeux expiré par le patient, de l'appareil anesthésiant, ladite cartouche étant remplacée par une autre cartouche identique lorsqu'une percée de gaz se produit, et comprenant les étapes de captage dudit courant gazeux expiré par le patient, quittant ledit orifice de sortie de la cartouche, pour déterminer la présence dans ledit courant gazeux expiré par le patient de ladite percée de gaz et l'actionnement d'une alarme détectable pour indiquer que ledit lit de matière du type silicalite est saturé et nécessite un remplacement.

8. Procédé suivant la revendication 7, dans lequel le lit de matière adsorbante saturé est régénéré dans la cartouche, et le gaz de purge est passé à travers ladite cartouche par l'orifice d'admission et l'orifice de sortie pour désorber l'agent anesthésiant organique de la matière du type silicalite.

9. Procédé suivant la revendication 8, dans lequel la cartouche est chauffée pour élever la température de la matière adsorbante au cours de la régénération afin de faciliter la désorption de l'agent anesthésiant organique.

10. Procédé suivant l'une quelconque des revendications précédentes, dans lequel le gaz de purge consiste en azote ou en air.

11. Procédé suivant la revendication 7, dans lequel le courant gazeux expiré par le patient, provenant d'un ou plusieurs patients, comprend au moins deux agents anesthésiants organiques différents, la matière adsorbante adsorbant simultanément lesdits agents anesthésiants organiques différents qui sont désorbés par le gaz de purge et condensés à partir de ce gaz sous une forme liquide isolée, ledit liquide isolé étant purifié par distillation fractionnée dudit liquide isolé pour séparer les agents anesthésiants organiques respectifs en agents anesthésiants organiques distincts.

12. Procédé suivant la revendication 11, dans lequel deux agents anesthésiants différents sont séparés par distillation fractionnée, lesdits agents anesthésiants consistant en éther de 1,1,2-trifluoro-2-chloréthyle et difluorométhyle, et en éther de 1-chloro-2,2,2-trifluoréthyle et difluorométhyle.

13. Procédé suivant la revendication 11 ou 12, dans lequel la matière adsorbante saturée est chauffée à des températures comprises dans l'intervalle de 30°C à 150°C, le gaz inerte de purge consistant en azote ou en air.

14. Procédé suivant la revendication 11, 12 ou 13, dans lequel le courant gazeux de purge avec l'agent anesthésiant désorbé est passé à travers un condenseur fonctionnant à une température suffisamment basse pour séparer dudit courant gazeux de purge lesdits agents anesthésiants condensés sous forme liquide.

15. Procédé suivant la revendication 14, dans lequel le liquide condensé est soumis à une distillation fractionnée en plusieurs étapes pour la purification des agents anesthésiants.

16. Procédé suivant la revendication 15, dans lequel le gaz de purge est chauffé à la température comprise dans l'intervalle de 30°C à 150°C, la matière du type silicalite étant chauffée par le gaz de purge consistant en azote au cours de la désorption des agents anesthésiants.

17. Procédé suivant la revendication 16, dans lequel la plage de températures à laquelle l'adsorbant et le gaz sont chauffés va de 30°C à 150°C.

18. Cartouche destinée à être utilisée dans le procédé suivant l'une quelconque des revendications 1 à 17, pour l'adsorption d'hydrocarbures halogénés à partir d'un courant gazeux passé à travers ladite cartouche, ladite cartouche possédant une paroi latérale périphérique, une première paroi terminale avec un orifice d'admission et une seconde paroi terminale avec un orifice de sortie, un premier filtre espacé de ladite première paroi terminale et obturant une première extrémité de la cartouche, un second filtre espacé de ladite seconde paroi terminale et obturant une seconde extrémité de la cartouche, une matière adsorbante granulaire consistant en un tamis moléculaire hydrophobe étant tassée dans ladite cartouche entre lesdits premier et second filtres, ladite matière adsorbante consistant essentiellement en une matière zéolitique à haute teneur en silice ayant un rapport SiO₂/Al₂O₃ compris dans l'intervalle de 12 à 50, ledit tamis moléculaire adsorbant ayant des diamètres des pores suffisamment grands pour permettre aux molécules des hydrocarbures halogénés de le traverser et d'être sélectivement adsorbées dans les grandes cavités internes du réseau cristallin, les hydrocarbures halogénés étant ainsi séparés sélectivement du courant gazeux, caractérisée en ce que lesdits premier et second filtres sont constitués par des grilles à maille fine ayant un diamètre des mailles permettant de retenir ladite matière granulaire dans ladite cartouche, et des moyens élastiques sont fournis pour pousser l'une desdites grilles vers l'autre afin de comprimer la matière granulaire entre les grilles.

19. Cartouche suivant la revendication 18, dans laquelle la matière adsorbante possède un diamètre des pores d'approximativement 0,6 nm (6 amgströms).

20. Cartouche suivant la revendication 18 ou 19, dont les parois sont formées d'un métal résistant à la corrosion.

21. Appareil anesthésiant dans lequel l'orifice d'admission d'une cartouche suivant la revendication 18, 19 ou 20 est connecté à un orifice d'évacuation de l'appareil anesthésiant pour faire passer ainsi le courant gazeux expiré par le patient dudit appareil anesthésiant à travers ladite cartouche et provoquer ainsi l'adsorption des gaz anesthésiants provenant du courant gazeux expiré par le patient.

22. Appareil pour régénérer la cartouche de silicalite suivant la revendication 18, 19 ou 20, comprenant un moyen pour connecter un conduit d'alimentation d'azote gazeux ou d'air à l'orifice d'admission, un moyen pour chauffer l'azote gazeux ou l'air dans ledit conduit d'alimentation à une température comprise dans l'intervalle de 30°C à 150°C, un moyen pour connecter un conduit de sortie à l'orifice de sortie et un moyen pour mesurer la température dans ledit conduit de sortie de l'azote gazeux enrichi en agent anesthésiant désorbé.

23. Appareil suivant la revendication 22, dans lequel le conduit de sortie est connecté à un condenseur fonctionnant à une température suffisamment basse pour séparer sous forme liquide les agents anesthésiants du courant d'azote gazeux, et comprenant un moyen pour recueillir le condensat liquide provenant du condenseur.
